# EUROPEAN PATENT APPLICATION

(11) **EP 4 495 878 A1**
(43) Date of publication of application: **22.01.2025**
(21) Application number: 23306243.9
(22) Date of filing: 19.07.2023
(51) Int. Cl.: G06T 7/00, A61B 6/00, G06V 10/25

(54) **ASSESSING PLAQUE BURDEN IN A PERIPHERAL BLOOD VESSEL OF A SUBJECT**

(71) Applicant: Koninklijke Philips N.V., 5656 AG Eindhoven (NL)
(72) Inventor: ROUET, Laurence, 5656 EINDHOVEN (NL); COLLET BILLON, Antoine, 5656 EINDHOVEN (NL); FLORKOW, Mateusz, 5656 EINDHOVEN (NL); ESLAMI, Parastou, 5656 EINDHOVEN (NL); SANCHEZ GONZALEZ, Javier, 5656 EINDHOVEN (NL); NAVARRO GUZMAN, Alvaro, 5656 EINDHOVEN (NL); PRABHU, David, 5656 EINDHOVEN (NL); ENTREKIN, Robert, 5656 EINDHOVEN (NL)
(74) Representative: Philips Intellectual Property & Standards

(57) **Abstract**

A system and method are provided for assessing plaque burden in a blood vessel of a subject. The method includes acquiring a three-dimensional, 3D, ultrasound image of the vessel using non-invasive ultrasound imaging (S511); performing 3D vessel segmentation of the vessel in the ultrasound image using a vessel segmentation machine learning model to identify borders of the vessel (S512); performing 3D plaque segmentation of the plaque in the ultrasound image using a plaque segmentation machine learning model to identify plaque within the borders of the vessel (S513); detecting a centerline of the vessel based on the 3D vessel segmentation of the vessel (S516); determining volume of the plaque within the borders of the vessel based on the 3D vessel segmentation (S517); and determining local thickness of the plaque in at least one cross-section of the vessel relative to the detected centerline based on the 3D vessel segmentation and the 3D plaque segmentation (S518).

## Description

### FIELD OF THE INVENTION

The invention relates to a system and method for assessing plaque burden in a peripheral blood vessel of a subject.

### BACKGROUND OF THE INVENTION

Cardiovascular disease (CVD), which is the most common cause of death in the United States, is caused by atherosclerosis and is best treated with early detection. Gold-standard methods for CVD assessment and risk stratification, such as CT angiography, are expensive and not easily accessible. Also, CT angiography exposes the clinicians and patients to ionizing radiation and the patient to nephrotoxic contrast agents.

In comparison, ultrasound imaging provides a non-invasive, inexpensive, easily accessible, and non-ionizing approach to CVD assessment and risk stratification. However, direct imaging of coronary arteries, for example, using non-invasive ultrasound can be challenging, and is subject to the motion of the heart and the skill of the sonographer.

As an alternative to direct ultrasound imaging of the coronary arteries, studies have demonstrated CVD plaque burden may be assessed indirectly via quantification of the plaque burden in peripheral arteries, such as the carotid artery. However, assessment of peripheral artery plaque burden with current software approaches is manually intensive and time-consuming. Therefore, what is needed is an automated technique for quickly, accurately and consistently measuring plaque burden (e.g., volume, thicknesses, etc.) in peripheral arteries using three-dimensional (3D) ultrasound images.

### SUMMARY OF THE INVENTION

The invention is defined by the independent claims. The dependent claims define advantageous embodiments.

In a representative embodiment, a system is provided for assessing plaque burden in a vessel of a subject. The system includes a display configured to display a three-dimensional (3D) ultrasound image of the vessel acquired using non-invasive ultrasound imaging; a user interface; a processor in communication with the display and the user interface, which processor may include a non-transitory memory storing instructions. The processor is configured to receive the 3D ultrasound image of the vessel; perform 3D vessel segmentation of the vessel in the 3D ultrasound image using a vessel segmentation machine learning model to identify borders of the vessel; perform 3D plaque segmentation of the plaque in the ultrasound image using a plaque segmentation machine learning model to identify plaque within the borders of the vessel; detect a centerline of the vessel based on the vessel segmentation; determine volume of the plaque within the borders of the vessel based on the 3D plaque segmentation; and determine local thickness of the plaque in at least one cross-section of the vessel relative to the detected centerline of the vessel based on the 3D vessel segmentation and the 3D plaque segmentation.

In another representative embodiment, a method is provided for assessing plaque burden in a vessel of a subject. The method includes acquiring a 3D ultrasound image of the vessel using non-invasive ultrasound imaging; performing 3D vessel segmentation of the vessel in the ultrasound image using a vessel segmentation machine learning model to identify borders of the vessel; performing 3D plaque segmentation of the plaque in the ultrasound image using a plaque segmentation machine learning model to identify plaque within the borders of the vessel; detecting a centerline of the vessel based on the 3D vessel segmentation of the vessel; determining volume of the plaque within the borders of the vessel based on the 3D plaque segmentation; and determining local thickness of the plaque in at least one cross-section of the vessel relative to the detected centerline based on the 3D vessel segmentation and the 3D plaque segmentation.

In another representative embodiment, a computer program product (e.g. software that can be downloaded from a server via e.g. the internet, or stored on a non-transitory computer readable medium) comprises instructions for assessing plaque burden in a vessel of a subject. When executed by a processor, the instructions cause the processor to carry out the above method, i.e. receive a 3D ultrasound image of the vessel acquired using non-invasive ultrasound imaging; perform 3D vessel segmentation of the vessel in the 3D ultrasound image a vessel segmentation machine learning model to identify borders of the vessel; perform 3D plaque segmentation of the plaque in the ultrasound image using a plaque segmentation machine learning model to identify plaque within the borders of the vessel; implement editing instructions for editing at least one of the 3D vessel segmentation or the 3D plaque segmentation to provide a corrected 3D vessel segmentation of the vessel and/or a corrected 3D plaque segmentation of the plaque; detect a centerline of the vessel based on the corrected 3D vessel segmentation; determine volume of the plaque within the borders of the vessel based on the corrected 3D plaque segmentation; and determine local plaque thickness of the plaque in at least one cross-section of the vessel relative to the detected centerline based on the 3D vessel segmentation and the 3D plaque segmentation.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a simplified block diagram of a system for assessing plaque burden in a vessel of a subject, according to a representative embodiment.
Fig. 2 is an image of a bifurcated vessel with detected centerlines, according to a representative embodiment.
Fig. 3A is an ultrasound image showing the cross-section of a vessel with an initial vessel border and plaque acquired by 3D vessel segmentation and 3D plaque segmentation, according to a representative embodiment.
Fig. 3B is an ultrasound image showing the cross-section of the vessel with a corrected (modified) vessel border, according to a representative embodiment.
Fig. 4A is an ultrasound image showing the cross-section of a vessel and plaque with an initial plaque border acquired by 3D vessel segmentation and 3D plaque segmentation, according to a representative embodiment.
Fig. 4B is an ultrasound image showing the cross-section of the vessel with a corrected (modified) plaque border, according to a representative embodiment.
Fig. 5 is a flow diagram of a method for assessing plaque burden in a vessel of a subject, according to a representative embodiment.

### DETAILED DESCRIPTION OF EMBODIMENTS

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items. As used in the specification and appended claims, and in addition to their ordinary meanings, the terms "substantial" or "substantially" mean to within acceptable limits or degree. As used in the specification and the appended claims and in addition to its ordinary meaning, the term "approximately" means to within an acceptable limit or amount to one having ordinary skill in the art. For example, "substantially" or "approximately" the same means that one of ordinary skill in the art would consider the items being compared to be the same.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments described herein provide a system and method that provides fully automated assessment of peripheral artery atherosclerotic plaque burden using non-invasive, 3D ultrasound imaging, as opposed to two-dimensional (2D) ultrasound imaging. The embodiments provide an end-to-end deep learning model(s) for fully automated 3D (peripheral artery) vessel segmentation and 3D plaque segmentation, and bifurcation-compatible 3D vessel centerline detection, which facilitates plaque thickness measurements. Also, the vessel and plaque segmentation results may be edited. 3D quantification of plaque echogenicity and 3D visualization of results with color-coded plaque thickness are also supported.

According to the various embodiments, the need for manual interaction by the user is reduced since both 3D vessel segmentation and 3D plaque segmentation are performed for the entire image volume, as opposed to having to perform 2D vessel segmentation and 2D plaque segmentation of individual 2D cross-sections (frames) of the vessel. Therefore, the segmentations are spatially consistent and coherent throughout the vessel, for both vessel boundaries and plaque. In addition, the plaque segmentation correction process is expedited by enabling 3D editing by the user of the plaque segmentation results, as opposed to 2D frame-by-frame editing alone. The bifurcation-compatible vessel centerline detection enables automated detection of a main vessel, as well as vessel branches from the main vessel, thereby reducing the amount of manual interaction required to determine the plaque burden in different vessel branches, which may have different clinical significance. The various embodiments enable automated analysis of 3D plaque echogenicity measurements, as opposed to 2D plaque echogenicity evaluations. Also, 3D colormaps may be correlated with plaque burden severity (lumen narrowing, plaque area, plaque echogenicity), which enables quick identification of the vessel area with the most significant disease.

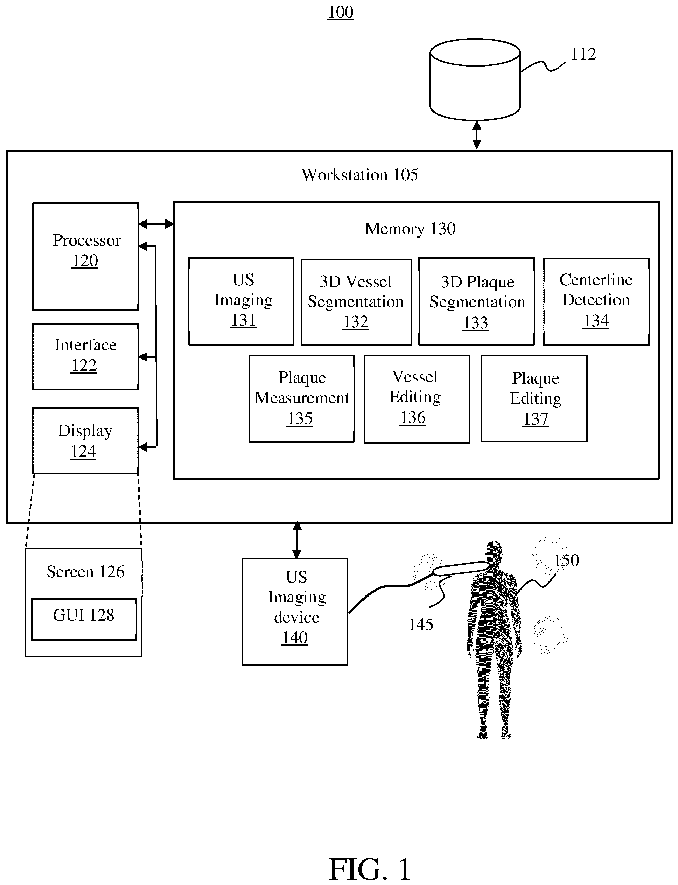

Fig. 1 is a simplified block diagram of a system for assessing atherosclerotic plaque burden in a vessel of a subject, according to a representative embodiment. The vessel may be a peripheral artery, such as the carotid or femoral arteries, which are relatively large, superficial and stationary, and therefore easier to image accurately with ultrasound, as compared to ultrasound imaging of the much smaller coronary arteries on the moving surface of the beating heart.

Referring to Fig. 1, system 100 includes a workstation 105 for implementing and/or managing the processes described herein with regard to assessing plaque burden in a vessel of interest in a subject (patient) 150 using 3D ultrasound images from an ultrasound imaging device 140. The workstation 105 includes processor 120, memory 130, a user interface 122 and a display 124. The processor 120 communicates with the ultrasound imaging device 140 through an imaging interface (not shown). The ultrasound imaging device 140 includes an ultrasound transducer probe 145 operable by a user to obtain 3D ultrasound images of plaque in a vessel of the subject 150, such as the carotid artery, which has been shown to correlate with the cardiovascular risk of a patient.

The memory 130 stores instructions executable by the processor 120. When executed, the instructions cause the processor 120 to implement one or more processes for performing a plaque burden assessment the subject 150 using ultrasound images acquired by the ultrasound imaging device 140. The ultrasound images may be provided from the ultrasound imaging device 140 in real-time or near real-time during the scanning procedure, or may be retrieved from storage (e.g., database 112) following the scanning procedure. For purposes of illustration, the memory 130 is shown to include software modules, each of which includes the instructions, executable by the processor 120, corresponding to an associated capability of the system 100.

The processor 120 is representative of one or more processing devices, and may be implemented by a general purpose computer, a central processing unit (CPU), a digital signal processor (DSP), a graphical processing unit (GPU), a computer processor, a microprocessor, a state machine, programmable logic device, field programmable gate arrays (FPGAs), application specific integrated circuits (ASICs), or combinations thereof, using any combination of hardware, software, firmware, hardwired logic circuits, or combinations thereof. Any processor or processing unit herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 130 is representative of one or more memories, and may include main memory and/or static memory, where such memories may communicate with each other and the processor 120 via one or more buses. The memory 130 may be implemented by any number, type and combination of random-access memory (RAM) and read-only memory (ROM), for example, and may store various types of information, such as software algorithms, artificial intelligence (AI) machine learning models, and computer programs, all of which are executable by the processor 120. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

The system 100 may also include database 112 for storing information that may be used by the various software modules of the memory 130. For example, the database 112 may include image data from previously obtained ultrasound images of the subject 150 and/or of other similarly situated subjects. The stored image data may be used for training an AI machine learning model, such as a 3D UNet model and/or a neural network model, for example, as discussed below. The database 112 may be implemented by any number, type and combination of RAM and ROM, for example. The various types of ROM and RAM may include any number, type and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The database 112 comprises tangible storage mediums for storing data and executable software instructions and may be non-transitory during the time data and software instructions are stored therein. The database 112 may be secure and/or encrypted, or unsecure and/or unencrypted. For purposes of illustration, the database 112 is shown as a separate storage medium, although it is understood that it may be combined with and/or included in the memory 130, without departing from the scope of the present teachings.

The processor 120 may include or have access to an artificial intelligence (AI) engine, which may be implemented as software that provides artificial intelligence (e.g., UNet models, neutral network models) and applies machine learning described herein. The AI engine may reside in any of various components in addition to or other than the processor 120, such as the memory 130, an external server, and/or the cloud, for example. When the AI engine is implemented in a cloud (not shown), such as at a data center, for example, the AI engine may be connected to the processor 120 via the internet or other communication network using one or more wired and/or wireless connection(s).

The user interface 122 is configured to provide information and data output by the processor 120, the memory 130 and/or the ultrasound imaging device 140 to the user and/or for receiving information and data input by the user. That is, the user interface 122 enables the user to enter data and to control or manipulate aspects of the processes described herein, and also enables the processor 120 to indicate the effects of the user's input, which may include control or manipulation of the ultrasound transducer probe 145. All or a portion of the user interface 122 may be implemented by a graphical user interface (GUI), such as GUI 128 viewable on the display 124, discussed below. The user interface 122 may include one or more interface devices, such as a mouse, a keyboard, a trackball, a joystick, a microphone, a video camera, a touchpad, a touchscreen, voice or gesture recognition captured by a microphone or video camera, for example.

The display 124 may be a monitor such as a computer monitor, a television, a liquid crystal display (LCD), an organic light emitting diode (OLED) display, a flat panel display, a solid-state display, or a cathode ray tube (CRT) display, or an electronic whiteboard, for example. The display 124 includes a screen 126 for viewing ultrasound images of the subject 150, along with various features described herein to communicate to the user volumes of plaque, for example, indicated in the images, as well as the GUI 128 to enable the user to interact with the displayed images and features. In an embodiment, the ultrasound imaging device 140 may include a separate dedicated display for acquiring the ultrasound images, where dedicated display is also represented by the display 124.

Referring to the memory 130, the various modules therein store sets of data and instructions executable by the processor 120 to perform the plaque burden evaluation, as mentioned above. Ultrasound imaging module 131 is configured to receive and process 3D ultrasound images of the vessel of interest in the subject 150. The 3D ultrasound images are acquired non-invasively by the ultrasound imaging device 140 via the ultrasound transducer probe 145, and may be received from the ultrasound imaging device 140 in real-time or near real-time during a contemporaneous imaging session of the subject 150. Alternatively, or in addition, the 3D ultrasound images may be previously acquired images, obtained during previous imaging session(s), which have been retrieved from storage (e.g., database 112). The 3D ultrasound images may be displayed on the display 124. The display of real-time images, in particular, enables the user to visualize the anatomy of the subject 150 while manipulating the ultrasound transducer probe 145.

Vessel segmentation module 132 is configured to receive a 3D ultrasound image of the vessel of interest from the ultrasound imaging module 131, and to perform 3D vessel segmentation of the vessel to identify borders of the vessel. The 3D vessel segmentation may be performed using a vessel segmentation machine learning model, for example, which may include various known vessel segmentation machine learning models. The 3D vessel segmentation enables more efficient segmentation of the complete extent of the vessel than conventional 2D segmentation techniques. The vessel segmentation machine learning model may be defined by a deep learning neural network, such as a convolutional neural network (CNN), e.g., UNet, an artificial neural network (ANN), or a recurrent neural network (RNN), for example. In an embodiment, at least a portion of the 3D vessel segmentation may be performed manually by the user via the user interface 122, and/or the results of the 3D vessel segmentation may be edited by the user via the user interface 122, as discussed below.

In an embodiment, as pre-processing, the 3D vessel segmentation may include performing normalization of the 3D ultrasound image (e.g., z-score normalization), and resampling the 3D ultrasound image to a higher resolution (e.g., about 0.2 x 0.2 x 0.2 mm³). The vessel segmentation machine learning model outputs a 3D vessel mask (image) as a 3D model using the normalized and resampled 3D ultrasound image as input. For example, the 3D vessel mask may be a binary vessel mask, in which "1" corresponds to the vessel (including vessel border and interior of the vessel) and "0" corresponds to background (including outside of the vessel). The vessel mask fits the borders of the vessel and includes only an interior volume of the vessel as defined by the borders of the vessel identified.

As post-processing of the 3D model, the 3D vessel segmentation includes performing connected component analysis to facilitate analysis of the vessel. Generally, connected component analysis includes uniquely labelling a binary mask into separate components based on connectivity of the components. In 3D, separation of the components may be defined on 6-connectivity (faces connected), 18-connectivity (edges connected) or 26-connectivity (nodes connected). The connected component analysis may include, for example, extracting the largest component of the resampled 3D ultrasound image to remove potential sparse false positive segmentations. In addition, image intensity-guided ballooning of the vessel mask may be performed as post-processing to further fit the vessel borders. Ballooning refers to a user interface technique of editing or correcting the position of a vessel (and/or plaque) boundary. The balloon, as defined by the user, "pushes" against the boundary and forces it to move to a different position. The boundary may "push back" in response, depending on the brightness of the pixels around it. Ballooning is generally based on the idea that vascular walls produce strong echoes that get written in the ultrasound image as bright pixels, whereas the blood-filled interior of the vessel (and some types of acoustic noise) generally produce weak echoes, and are black or dark pixels in the image. The centerline of the vessel may be detected using the vessel mask, as discussed below with reference to centerline detection module 134.

The vessel segmentation module 132 is further configured to derive a polygonal mesh, comprising vertices and edges, from the vessel mask. The meshes may be derived during post-processing using a known marching cube algorithm, for example. The vessel borders are represented by the polygonal mesh as derived by the vessel segmentation module 132.

Plaque segmentation module 133 is configured to receive the 3D ultrasound image of the vessel from the ultrasound imaging module 131 and the vessel mask from the vessel segmentation module 132, and to perform 3D plaque segmentation of the plaque in the ultrasound image to identify plaque within the vessel borders. The 3D plaque segmentation may be performed using a plaque segmentation machine learning model, for example, which may include various known plaque segmentation machine learning models. The 3D plaque segmentation enables more efficient segmentation of the complete volume of the plaque in the vessel than conventional 2D segmentation techniques. The plaque segmentation model may be defined by a deep learning neural network, such as a CNN (e.g., UNet), an ANN, or an RNN, for example. It is understood that the vessel segmentation machine learning model and the plaque segmentation machine learning model may be different machine learning models (as shown), respectively, or may be implemented by a single machine learning model trained for both types of segmentations, without departing from the scope of the present teachings. In an embodiment, at least a portion of the 3D plaque segmentation may be performed manually by the user via the user interface 122, and/or the results of the 3D plaque segmentation may be edited by the user via the user interface 122, as discussed below.

In an embodiment, as pre-processing, the 3D plaque segmentation includes performing normalization of the 3D ultrasound image (e.g., z-score normalization), and resampling the 3D ultrasound image to a higher resolution (e.g., about 0.18 x 0.18 x 0.18mm³). The plaque segmentation machine learning model outputs a 3D plaque mask (image) as a 3D model using the normalized and resampled 3D ultrasound image and the 3D vessel mask as input. As post-processing, the 3D plaque segmentation includes performing connected component analysis to facilitate analysis of the plaque. For example, the connected component analysis may include color-coding the different components within the areas of segmented plaque. The plaque segmentation module 133 is further configured to derive a polygonal mesh, comprising vertices and edges, from the plaque mask. The meshes may be derived during post-processing using a known marching cube algorithm, for example. The plaque borders are represented by the polygonal mesh as derived by the plaque segmentation module 133.

The vessel and plaque segmentation machine learning models may be applied sequentially. For example, with regard to resampling, the 3D ultrasound image is first resampled for the 3D vessel segmentation, and then resampled again for the 3D plaque segmentation. This approach allows for interaction with (and editing of) the vessel mask by the vessel segmentation machine learning model before applying the plaque segmentation machine learning model, thereby assuring correct 3D vessel segmentation followed by coherent 3D plaque segmentation that matches the vessel borders as determined by the 3D vessel segmentation. The interaction with the vessel mask may be performed with a 3D deformable model.

In various embodiments, all or part of the processes provided by the vessel and plaque segmentation machine learning models may be implemented by one or more AI engines, for example, mentioned above. The vessel and plaque segmentation machine learning models may be trained using retrospective training ultrasound images of peripheral vessels in multiple patients, and ground truth data indicating vessel borders and plaque borders that were associated with the training ultrasound images, respectively. The segmented vessels and segmented plaque are associated with corresponding vessel borders and plaque volumes. The training data may be stored in one or more databases, such as the database 112, for example, and may be annotated by clinical experts.

Centerline detection module 134 is configured to detect centerlines of the vessel based on the vessel borders determined by the vessel segmentation module 132 (e.g., output by the vessel segmentation machine learning model). In an embodiment, the centerline of the vessel may be detected using an image processing technique, such as a fast-marching algorithm (e.g., Eikonal equation), for example. Generally, the fast-marching algorithm solves a wave propagation equation, where the refractive index of the wave propagation equation is derived from a distance map to the vessel border. The fast-marching algorithm receives pairs of points in the segmented vessel as input, and outputs the minimal geodesic path between the pairs of points (i.e., the Fermat principle), respectively. For example, for a carotid artery, the input pairs of points include {center of proximal common carotid, center of distal internal carotid} and {center of proximal common carotid, center of distal external carotid}.

The pairs of points may be endpoints of pieces of the centerline in the vessel. For example, the pairs of points may be determined by calculating transverse cuts along a longitudinal axis of the vessel (e.g., volume z-axis or volume x-axis depending on the orientation of the ultrasound transducer probe 145), tracking 2D connected components of the transverse cuts, and identifying locations of any bifurcations and the direction of the ultrasound scan providing the 3D ultrasound image for division from the main vessel to multiple vessel branches. Determining the pairs of points further includes determining the centers of gravity of the connected components, respectively, to provide estimates of the pieces of the centerline (including any vessel branches). The endpoints of the estimated centerline pieces are input as the pairs of points to the fast-marching algorithm in a 3D vessel potential map derived from the 3D vessel segmentation. With the right 3D vessel potential map, the fast-marching algorithm produces centerlines that merge deep in the carotid arteries. However, in carotid arteries, clinical focus is set on the internal-common branch, where the external carotid artery is considered to be a secondary branch of lesser importance clinically. Hence, after applying the fast-marching algorithm, the centerline of the external carotid artery may be corrected by forcing an early merge of the centerline of the external carotid artery with the centerline of the internal-common carotid branch when the centerline of the external carotid artery gets close enough. This provides an asymmetrical result better representative of the anatomy.

The process of detecting the centerlines of the vessel results in automatically detecting a centerline in a main vessel, as well as centerlines in any vessel branches from the main vessel. Generally, the centerlines of the vessel branches are required to provide a natural coordinate system, such as a curvilinear coordinate system and a local 2D axis system perpendicular to the centerline. The natural coordinate system is needed for 2D quantification of the plaque, such as measuring local plaque thickness, as discussed below.

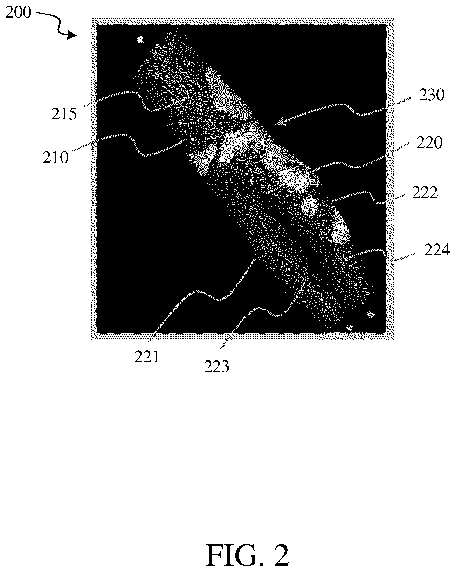

Fig. 2 is an image of a bifurcated vessel with detected centerlines, according to a representative embodiment. Referring to Fig. 2, vessel 200 includes a main vessel 210 and vessel branches 221 and 222 following bifurcation 220. Detected centerline 215 longitudinally follows the main vessel 210, and the detected centerlines 223 and 224 longitudinally follow the vessels branches 221 and 222, respectively. The plaque 230 shown in the vessel 200 is measured relative to the detected centerlines 215, 223 and 224, as discussed below.

Referring again to Fig. 1, plaque measurement module 135 is configured to determine the 3D volume and 2D thicknesses of the plaque within the borders of the vessel. The 3D volume and thicknesses of the plaque are used for CVD assessment and risk stratification of the subject 150. Generally, the greater the 3D volume and/or thicknesses of the plaque, as determined by the plaque measurement module 135, the more advanced the CVD in the subject 150. The 3D volume and 2D thicknesses of the plaque may be assessed based on overall values of the plaque and/or percentages of the plaque relative to the 3D volume and/or 2D cross-sections of the vessel.

The plaque measurement module 135 is configured to determine the 3D volume of the plaque based on the 3D plaque segmentation. In particular, the 3D volume of the plaque is calculated mathematically from the coordinates of the vertices and edges of the mesh derived by the plaque segmentation module 133, discussed above.

The plaque measurement module 135 is further configured to determine 2D local thicknesses of the plaque in cross-sections (slices) of the vessels relative to the detected centerline(s) of the vessel. The plaque measurement module 135 may also optionally determine 2D plaque areas of these cross-sections. The detected centerline is used to define a local 2D plane perpendicular to the centerline, and the local thickness of the plaque is determined as the maximal thickness of the plaque in the local 2D plane. Multiple local thicknesses of the plaque may be determined in multiple local 2D planes defined by the detected centerline. As mentioned above, the detected centerline of the vessel provides a natural coordinate system, such as a curvilinear coordinate system, and a local 2D axis system perpendicular to the detected centerline, for identifying the local 2D planes. The local thicknesses provide 2D quantification of the plaque, which may also be used for determining the plaque area, stenosis, and other measurements that implicitly require local orthogonality to the vessel borders. The stenosis, in particular, is the percentage reduction in diameter or area of the vessel lumen at any cross-section due to the thickness of the plaque at the same cross-section, thus hindering the flow of blood.

In various embodiments, the system 100 enables optional editing of the 3D vessel segmentation and the 3D plaque segmentation by the user via the user interface 122. In some cases, image artifacts, such as shadows from calcification and clutter noise, degrade the performances of the automatic segmentation of 3D vessel and plaque borders to a point where manual editing by the user is desirable to obtain accurate quantification results. Generally, conventional techniques for correcting 2D planar contours are very cumbersome and ill-suited to correct 3D segmentation models by nature, since hundreds of 2D planes or slices may be required to accurately represent the 3D volume containing the vessel. Therefore, intrinsically 3D editing schemes for correcting the 3D vessel and plaque segmentations, from which the vessel and plaque borders have been derived, may be applied to the 3D ultrasound image of the vessel. The corrections to the 3D vessel and plaque segmentations may be input by the user in any plane orientation, and may extend to a 3D neighborhood at a scale that is chosen by the user. For the 3D plaque segmentation, there is also a correction applied in the 3D view. That is, the plaque may be constituted of multiple individual components (not connected), and the correction includes deleting one of the spurious components (or all except for one of the spurious components).

In system 100, vessel editing module 136 enables editing of the 3D vessel segmentation and plaque editing module 137 enables editing of the 3D plaque segmentation. That is, the vessel editing module 136 is configured to implement editing instructions received from the user via the user interface 122 for correcting the 3D vessel segmentation to provide a corrected vessel segmentation of the vessel, and the plaque editing module 137 is configured to implement editing instructions received from the user via the user interface 122 for correcting the 3D plaque segmentation to provide a corrected plaque segmentation of the vessel. In this case, the centerline detection by the centerline detection module 134 is based on the corrected vessel segmentation, and the plaque thickness determinations by the plaque measurement module 135 are made for the corrected plaque segmentation within the borders of the corrected vessel borders relative to the corrected centerline of the vessel.

The editing instructions received by the vessel editing module 136 for editing the 3D vessel segmentation may include input from the user interface 122 indicating points to which the surface of the border of the vessel is to be relocated. The points may be located inside or outside the initial vessel border to indicate that a smaller or larger cross-sectional area is desired, respectively. For example, the input may include touches to a touch screen or point clicks from a mouse or other input device on the 3D ultrasound image using the GUI 128. In response, the vessel editing module 136 implements the editing instructions for correcting the 3D vessel segmentation by relocating (moving) the surface of the vessel border to the indicated points to provide the relocated surface.

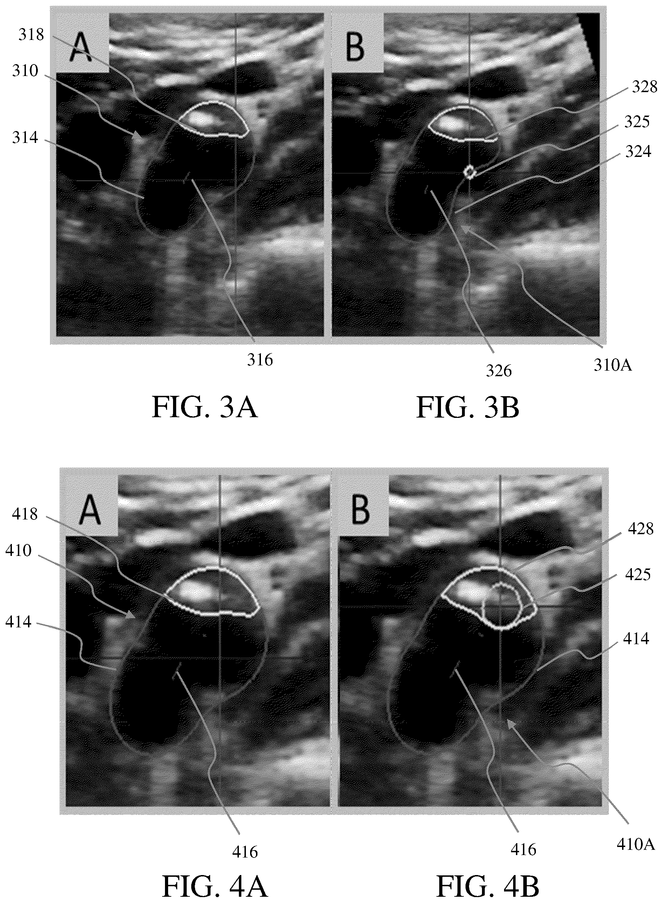

Fig. 3A is an ultrasound image showing the cross-section of a vessel with an initial vessel border and plaque acquired by 3D vessel segmentation and 3D plaque segmentation, according to a representative embodiment. Referring to Fig. 3A, the vessel 310 is shown following 3D vessel and plaque segmentation with an initial vessel border 314, an initial centerline 316 in the lumen of the vessel 310, and an initial plaque border 318 along a portion of the initial vessel border 314.

Fig. 3B is an ultrasound image showing the cross-section of the vessel with a corrected (modified) vessel border, according to a representative embodiment. Referring to Fig. 3B, a corrected vessel 310A is shown in response to editing instructions which established an anchor point 325 on the initial vessel border 314 inwardly to establish a corrected vessel border 324. The corrected vessel border 324 results in a corrected centerline 326, as well. The shape of the initial plaque border 318 also shifts slightly in response to the adjustment represented by the corrected vessel border 324, resulting in a corrected plaque border 328.

The amount of change to the initial vessel border 314 gradually decreases as the distance from the anchor point 325 (e.g., user click) along the vessel border 314 line increases, until original surface of the initial vessel border 314 is not changed. For example, in Figs. 3A and 3B, the left side of the corrected vessel border 324 is unchanged in comparison to the left side of the initial vessel border 314 due to its relatively large distance from the anchor point 325. Since the shape of the vessel 310 is smooth, corrections work at a scale that preserves that smooth nature, while the extent of the change is determined by the distance from the anchor point 325 to the surface. In an embodiment, the extent of change may be previewed on the display 124 by a rendered vessel mesh by means of colorization, for example.

The editing instructions received by the plaque editing module 137 for editing the 3D plaque segmentation may include input from the user interface 122 indicating a selected radius of a virtual balloon within a region of plaque provided by the 3D plaque segmentation in the ultrasound image. For example, the input may include touches to a touch screen or point clicks from a mouse or other input device on the 3D ultrasound image using the GUI 128. In response, the plaque editing module 137 implements the editing instructions for correcting the 3D plaque segmentation by relocating (moving) the surface of the plaque border to the indicated points to match the selected radius of the virtual balloon. Alternatively, or in addition, the editing instructions received by the plaque editing module 137 may include input from the user interface 122 indicating one or more individual connected components (as determined by the connected component analysis in post processing) to be removed from among connected components, thereby altering a region of plaque provided by the 3D plaque segmentation in the ultrasound image.

Fig. 4A is an ultrasound image showing the cross-section of a vessel and plaque with an initial plaque border acquired by 3D vessel segmentation and 3D plaque segmentation, according to a representative embodiment. Referring Fig. 4A, the vessel 410 is shown following 3D vessel and plaque segmentation with an initial vessel border 414, an initial centerline 416 in the lumen of the vessel 410, and an initial plaque border 418 along a portion of the initial vessel border 414.

Fig. 4B is an ultrasound image showing the cross-section of the vessel with a corrected (modified) plaque border, according to a representative embodiment. Referring to Fig. 4B, a corrected vessel 410A is shown in response to editing instructions which established a virtual balloon 425 within the boundaries of the initial plaque border 418 to establish a corrected plaque border 428. Meanwhile, the shape of the initial vessel border 414 and the position of the initial centerline 416 are substantially unchanged by the modification indicated by the corrected plaque border 428. The plaque segmentation corrections are set by the virtual balloon 425, where the radius of the virtual balloon 425 is determined interactively by the distance between an initial input (e.g., initial screen touch or click) in any planar cut and a subsequent input. The user may increase the size of the virtual balloon 425 until it meets the initial plaque border 418 and then push it beyond the initial plaque border 418, which deforms accordingly to accommodate the shape of the expanding virtual balloon 425 to provide the corrected plaque border 428. The process works in reverse as well, where the size of the virtual balloon 425 may be interactively decreased. Since the corrections are interactive, the user instantly sees the results of the modifications to the virtual balloon 425 and their corresponding effects on the shape of the corrected plaque border 428 on the display 114 substantially in real-time.

The user may repeat modifications to the virtual balloon 425 and thus corrections to the corrected plaque border 428 as much as needed, either starting over or starting from the most recent shape. The user may also use the lumen representation of the plaque surface at the initial plaque border 418 or the corrected plaque border 428 (i.e., the surface of the complement of the plaque inside the vessel 410) to add plaque where it may have been missed by segmentation. The interior of the vessel 410 is made up of the lumen and the plaque, separated at the initial plaque border 418. Therefore, modifications to the plaque may be performed in a "plaque view" or a "lumen view," which complements the plaque view, knowing that the size of the vessel 410 will remain unchanged. Corrections to the plaque may be accomplished by pushing the lumen border of the inside vessel (which is not possible with plaque representation). For example, pushing the lumen away from the vessel wall effectively moves the plaque border 418 as well, thereby increasing the amount of plaque.

The virtual balloon 425 may have a sphere shape (e.g., a distance map, an implicit function where the sphere is a level set) that is driven by image features. Edges or ridges of the sphere define a geodesic distance in lieu of a Euclidean distance, and conform the sphere shape to that distance. The user may pick different settings at the user interface 122 to adjust the degree of geodesic adaption that is best suitable to the situation. When there is no such adaptation, the sphere shape of the virtual balloon 425 is a Euclidian sphere.

Under certain circumstances, the user may want to extend vessel branches identified during the plaque measurement process above. In ultrasound scans of a carotid artery, image quality quasi-systematically degrades moving distally from the ultrasound transducer probe 145. This is primarily because of the increasing depth, as well as hindrance by the subject's jaw. Therefore, such ultrasound images are not of clinical grade and the presence of plaque cannot be accurately ascertained. Also, these areas are generally excluded from training of the vessel and plaque segmentation machine learning (AI) models.

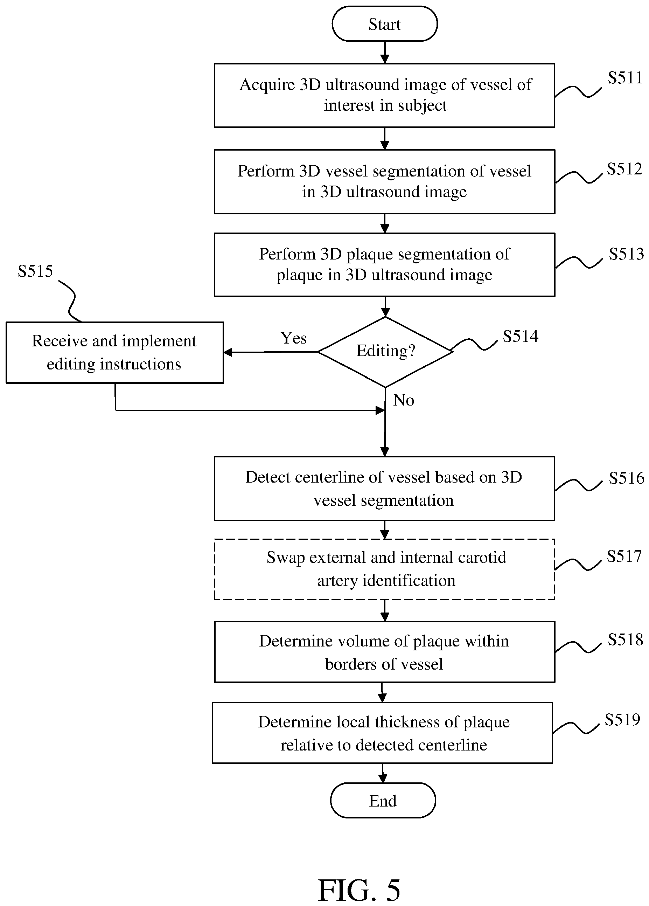

Fig. 5 is a flow diagram of a method for assessing plaque burden in a vessel of a subject, according to a representative embodiment. The method may be implemented using instructions stored in memory 130 and executable by the processor 120 in the system 100, for example, discussed above.

Referring to Fig. 5, a 3D ultrasound image of a vessel of interest in a subject is acquired in block S511. The 3D ultrasound image has been acquired using non-invasive ultrasound imaging, and may be received from an ultrasound imaging device or from a database of previously acquired 3D ultrasound images.

In block S512, 3D vessel segmentation of the vessel in the ultrasound image is performed using a vessel segmentation machine learning model to identify borders of the vessel. In block S513, 3D plaque segmentation of plaque in the ultrasound image is performed using a plaque segmentation machine learning model to identify borders of the plaque within the borders of the vessel. Each of the vessel segmentation model and the plaque segmentation model may be a neural network, such as UNet, an ANN, an RNN, or a CNN, for example. The neural network may be a deep learning neural network. Also, the vessel segmentation model and the plaque segmentation model may be implemented by a single, unified machine learning model or by separate, independent machine learning models, without departing from the scope of the present teachings.

In block S514, it is determined whether edits should be made to correct the 3D vessel segmentation and/or the 3D plaque segmentation. The determination may be made by a user based on visual inspection of the segmented ultrasound image. When it is determined that edits should be made to one or both of the 3D vessel segmentation or the 3D plaque segmentation (block S514: yes), the process proceeds to block S515. In block S515, editing instructions are received from the user interface and implemented for correcting the 3D vessel segmentation to provide a corrected vessel segmentation of the vessel and/or for correcting the 3D plaque segmentation to provide a corrected plaque segmentation of the plaque in the vessel. Generally, editing instructions for correcting the 3D vessel segmentation may include a location of an anchor point in the ultrasound image to which a portion of the vessel border is to be moved. Editing instructions for correcting the 3D plaque segmentation may include a location and selected radius of a virtual balloon placed within a region of the plaque in the ultrasound image, and adjusting a surface at the border of the region of plaque to match the location selected radius of the virtual balloon. The editing instructions for correcting the 3D vessel segmentation and the 3D plaque segmentation may be received from the user via a user interface, which may be include by a GUI, for example.

When it is determined in block S514 that no edits are needed to the 3D vessel segmentation or the 3D plaque segmentation (block S514: No), the process proceeds to block S516. In block S516, a centerline of the vessel is detected based on the 3D vessel segmentation of the vessel. This refers to either the initial 3D vessel segmentation provided by block S513 or, when determined, the corrected 3D vessel segmentation provided by block S515. Detecting the centerline of the vessel automatically detects centerlines of a main vessel and any vessel branches from the main vessel, thereby identifying any vessel branches as well. The vessel branches may be identified automatically, although the identification of the branches (e.g., internal versus external carotid artery) is checked by the user. The main centerline should go from the common carotid artery (before the branching) into the internal carotid artery. If the centerline goes from the common carotid artery to the external carotid artery, the user swaps the external and internal identifications, e.g., using a swap button on the user interface, in optional block S517, so that the initially identified external carotid artery is identified as the internal carotid artery, and vice versa.

In block S518, volume of the plaque within the borders of the vessel is determined based on the 3D plaque segmentation (which depends on the 3D vessel segmentation). As discussed above, the volume of the plaque may be determined by obtaining a mesh of the plaque and determining the volume of the plaque from the coordinates of vertices and edges of the mesh.

In block S519, local thicknesses of the plaque within the border of the vessel are determined relative to the detected centerline based on the 3D vessel segmentation and the 3D plaque segmentation. As discussed above, determining the local thicknesses includes identifying one or more cross-sections (slices) of the vessels relative to the detected centerline of the vessel (or multiple centerlines when the vessel includes branches), and measuring a maximal thickness of the plaque in the 2D plane defined by the cross-sections.

The volume and local thicknesses of the plaque are used for CVD assessment and risk stratification of the subject. Generally, the greater the volume and/or thicknesses of the plaque, the more advanced the CVD in the subject, thereby informing the treatment prescribed, if any. The volume and thicknesses of the plaque may be assessed based on overall values of the plaque and/or percentages of the plaque relative to the volume and/or the cross-sections of the vessel.

In accordance with various embodiments of the present disclosure, the methods described herein may be implemented using a hardware computer system that executes software programs stored on (non-transitory) storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although assessing plaque burden in a vessel has been described with reference to exemplary embodiments, it is understood that the words that have been used are words of description and illustration, rather than words of limitation. Changes may be made within the purview of the appended claims, as presently stated and as amended, without departing from the scope and spirit of the embodiments. Although assessing plaque burden has been described with reference to particular means, materials and embodiments, it is not intended to be limited to the particulars disclosed; rather assessing plaque burden extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A method of assessing plaque burden in a vessel of a subject, the method comprising:
receiving (S511) a three-dimensional, 3D, ultrasound image of the vessel using non-invasive ultrasound imaging;
performing (S512) 3D vessel segmentation of the vessel in the ultrasound image using a vessel segmentation machine learning model to identify borders of the vessel;
performing (S513) 3D plaque segmentation of the plaque in the ultrasound image using a plaque segmentation machine learning model to identify plaque within the borders of the vessel;
detecting (S516) a centerline of the vessel based on the 3D vessel segmentation of the vessel;
determining (S518) a volume of the plaque within the borders of the vessel based on the 3D plaque segmentation; and
determining (S519) a local thickness of the plaque in at least one cross-section of the vessel relative to the detected centerline based on the 3D vessel segmentation and the 3D plaque segmentation.

2. The method of claim 1, further comprising:
identifying at least one branch of the vessel based on the detected centerline.

3. The method of claim 1, further comprising:
calculating transverse cuts along a longitudinal axis of the vessel;
tracking two-dimensional (2D) connected components of the transverse cuts;
determining centers of gravity of the 2D connected components, respectively, to provide estimates of pieces of the centerline; and
applying a fast-marching algorithm to the estimated pieces of the centerline to detect the centerline of the vessel.

4. The method of claim 1, further comprising:
measuring stenosis of the vessel relative to the detected centerline of the vessel.

5. The method of claim 1, wherein each of the vessel segmentation machine learning model and the plaque segmentation machine learning model comprises a 3D UNet model.

6. The method of claim 1, wherein the step of performing the 3D plaque segmentation of the vessel comprises using a vessel mask that fits the borders of the vessel and the ultrasound image, wherein the vessel mask includes only an interior volume of the vessel as defined by the borders of the vessel identified by performing the 3D vessel segmentation.

7. The method of claim 1, further comprising:
implementing editing instructions for editing the 3D vessel segmentation to provide a corrected 3D vessel segmentation of the vessel; and
automatically updating the 3D plaque segmentation in response to the corrected 3D vessel segmentation of the vessel; and/or
implementing editing instructions for editing the 3D plaque segmentation to provide a corrected 3D plaque segmentation of the plaque,
wherein the volume of the plaque within the borders of the vessel is determined based on the corrected 3D plaque segmentation.

8. The method of claim 7, wherein the editing instructions for correcting the 3D vessel segmentation comprise a plurality of point clicks in the ultrasound image, received from the user interface, relocating a surface of the borders of the vessel inside or outside an original surface of the borders of the vessel, and
wherein implementing the editing instructions for correcting the 3D vessel segmentation comprises moving the borders of the vessel to the relocated surface.

9. The method of claim 7, wherein the editing instructions for editing the 3D plaque segmentation comprise a selected radius of a virtual balloon, received from the user interface, within a region of plaque provided by the 3D plaque segmentation in the ultrasound image,
wherein implementing the editing instructions for correcting the 3D plaque segmentation comprises moving a surface of the region of plaque to match the selected radius of the virtual balloon, and
wherein optionally the method further comprises:
implementing the editing instructions for correcting the 3D plaque segmentation by displaying in real-time on a display the moving of the surface of the region of plaque to match the selected radius of the virtual balloon.

10. The method of claim 1, wherein the local thickness of the plaque is determined relative to the detected centerline of the vessel using curvilinear coordinates of the detected centerline and a local 2D axis perpendicular to the detected centerline; or wherein the local thickness of the plaque in at least one cross-section of the vessel is determined as a maximal thickness of the plaque in a local 2D plane defined by the at least one cross-section.

11. The method of claim 1, further comprising:
resampling the ultrasound image to provide a first resampled 3D ultrasound image, and perform the 3D vessel segmentation of the vessel in the first resampled 3D ultrasound image; and
resampling the ultrasound image to provide a second resampled 3D ultrasound image, and perform the 3D plaque segmentation of the plaque in the second resampled 3D ultrasound image, wherein portions of the ultrasound image showing the plaque are resampled at a higher resolution than portions of the ultrasound image showing the vessel.

12. The method of claim 1, further comprising:
determining a 2D plaque area of the at least one cross-section of the vessel relative to the detected centerline based on the 3D vessel segmentation and the 3D plaque segmentation.

13. The method of any of the preceding claims, wherein the vessel comprises a carotid artery including a common carotid artery before branching of the carotid artery, the method further comprising:
checking that the centerline of the vessel goes from the common carotid artery into an internal carotid artery, and not an external carotid artery; and
when the centerline of the vessel goes from the common carotid artery into the external carotid artery, swapping identifications of the internal and external carotid arteries.

14. A system (100) for assessing plaque burden in a vessel of a subject, the system comprising:
a display (124) configured to display a three-dimensional (3D) ultrasound image of the vessel acquired using non-invasive ultrasound imaging;
a user interface (122); and
a processor (120) in communication with the display and the user interface, and configured to perform the method of any of claims 1 to 13.

15. A computer program product comprising instructions for assessing plaque burden in a vessel of a subject, that when executed by a processor, cause the processor to perform the method of any of claims 1 to 13.
